# EUROPEAN PATENT SPECIFICATION

(11) **EP 2 871 188 B1**
(45) Date of publication and mention of the grant of the patent: **19.04.2017**
(21) Application number: 13813495.2
(22) Date of filing: 05.07.2013
(51) Int. Cl.: C07H 15/04, A61K 31/706, A61P 37/06, G01N 30/88, C07H 17/08

(54) **METHOD FOR SEPARATING CYCLIC MACROLIDE COMPOUND**
VERFAHREN ZUR TRENNUNG EINER CYCLISCHEN MAKROLIDVERBINDUNG
PROCÉDÉ DE SÉPARATION DE COMPOSÉ MACROLIDE CYCLIQUE

(30) Priority: 06.07.2012 JP 2012152913
(43) Date of publication of application: 13.05.2015
(73) Proprietor: Godo Shusei Co., Ltd., Tokyo, 1048162 (JP)
(72) Inventor: TSUKUDA, Yuya, Matsudo-shi Chiba 271-0064 (JP); MURAMATSU, Keita, Matsudo-shi Chiba 271-0064 (JP); SASAKI, Hironori, Matsudo-shi Chiba 271-0064 (JP); NAKAMURA, Hirohide, Matsudo-shi Chiba 271-0064 (JP)
(74) Representative: Blodig, Wolfgang
(86) International application number: PCT/JP2013/068466
(87) International publication number: WO 2014/007362

(56) References cited:
- WO-A1-00/71546
- WO-A1-99/49863
- JP-A- 2005 315 668
- JP-A- 2005 531 603
- JP-A- 2007 516 951

## Description

### Technical Field

The present invention relates to a method for separation and purification of a cyclic macrolide compound, namely tacrolimus, that is useful as, for example, pharmaceutical products.

### Background Art

Among cyclic macrolide compounds, there are a large number of substances useful as pharmaceutical products such as antibiotics, for example, erythromycin, and immune-suppressing drugs, for example, tacrolimus. Many of the cyclic macrolide compounds are collected from cultured products of microorganisms . Since such cultured products contain a lot of analogous substances other than a target cyclic macrolide compound, various techniques for separating only the target compound have been reported.

For example, as a classic purification method of a natural compound, there is a technique in which a test solution is subjected to silica gel column chromatography in which silver is supported on the silica gel and a compound having a double bond in the skeleton and a compound without having a double bond are separated (for example, Non-Patent Literatures 1 and 2).

Tacrolimus and analogous substances thereof have a component having a double bond in the side chain skeleton and a component without having a double bond. Therefore, there is reported a technique in which silver is supported on a strongly acidic ion exchange resin and a mixture of tacrolimus and analogous substances thereof are passed through the resin to separate tacrolimus and the analogous substances (Patent Literature 1). Furthermore, there is reported a technique in which a mixture is adsorbed to a non-ionic adsorptive resin to separate tacrolimus and the analogous substances thereof by use of a silver ion-containing aqueous solvent, or the like (Patent Literature 2).

JP 2007/516951 discloses use of macrolide compounds for treating or preventing a pulmonary disease.

WO99/49863 A1 discloses macrolide compound preparations for oral administration.

JP 2005/531603 discloses 3-Descladinosyl-5-O-carbamoyl and 6-O-carbonoyl macrolide antibacterial agents.

JP 2005/315668 discloses a separation agent for an optical isomer suitable for high-speed liquid chromatography.

### Citation List

### Patent Literatures

Patent Literature 1: JP 3750527 B1
Patent Literature 2: JP 3925198 B1

### Non Patent Literatures

Non Patent Literature 1: Isolation and Generation of Substances, Otake, et al., p. 70 (University of Tokyo Press, 1976)
Non Patent Literature 2: J. Chromatograph. 69, 207 (1972)

### Summary of the Invention

### Problems to be solved by the Invention

However, although the techniques descried in the above patent literatures are effective for separation of a compound having a double bond in the side chain and a compound without having a double bond, they are not effective for separation of tacrolimus and a compound having a double bond in the side chain, which is the same as tacrolimus. In fact, it has been known that a tacrolimus-containing cultured product includes a compound having a double bond in the side chain in the same way as tacrolimus. In addition, since a silver compound is used, the techniques are required to conduct an operation of completely removing silver in a step of a post treatment. Consideration from an environmental point of view such as a waste liquid treatment is also necessary.

Therefore, the present invention is to provide a method capable of separating a target cyclic macrolide compound without using a silver compound even when a compound having a double bond similar to the target cyclic macrolide compound is mixed. Solution to Problem

Therefore, the present inventors have made various studies on techniques of separation and purification for a mixture comprising a cyclic macrolide compound such as tacrolimus and analogous compounds thereof as a target, and found that, when silica gel column chromatography using an asymmetry identifying agent-immobilized silica gel is employed, contrary to expectation, tacrolimus and several analogous substances thereof, which are not enantiomers, are efficiently separated and tacrolimus at a high purity can be produced, and the present invention was thus completed.

That is, the present invention is to provide the following [1] to [4].
[1] A method for separating a cyclic macrolide compound from a mixture comprising a cyclic macrolide compound and at least one analogous compound thereof, wherein the mixture is subj ected to silica gel column chromatography using an asymmetry identifying agent-immobilized silica gel, wherein the cyclic macrolide compound is tacrolimus and the asymmetry identifying agent-immobilized silica gel is a carbamate modified polysaccharide-immobilized silica gel or an optically active amine modified synthetic polymer-immobilized silica gel.
[2] The method according to [1], wherein the analogous compound thereof is at least one selected from the group consisting of ascomycin, FR-901156 and FR-900525.
[3] A method for producing a cyclic macrolide compound comprising at least one analogous compound thereof in the total amount of less than 0.27%, wherein a mixture comprising the cyclic macrolide compound and at least one analogous compound thereof is subjected to silica gel column chromatography using an asymmetry identifying agent-immobilized silica gel, wherein the cyclic macrolide compound is tacrolimus and the asymmetry identifying agent - immobilized silica gel is a carbamate modified polysaccharide-immobilized silica gel or an optically active amine modified synthetic polymer-immobilized silica gel.
[4] The method according to [3], wherein the analogous compound thereof is selected from the group consisting of ascomycin, FR-901156 and FR-900525.

### Effects of the Invention

According to the separation method of the present invention, the analogous compound, having differences with the target cyclic macrolide compound in terms of whether the analogous compound has double bonds in side chains or not and the analogous compound contains 1 or 2 larger or smaller number of carbon atoms, and the like, can be separated in a good yield. Even though a column to be used is a column essentially having an asymmetry identifying agent, it is utterly unexpected that the analogous compound which is slightly different from the cyclic macrolide compound in the number of carbon atoms can be separated.

### Brief Description of Drawings

Fig. 1 is a graph that illustrates a separation status by the separation method of Example 1.
Fig. 2 is a graph that illustrates a separation status by the separation method of Comparative Example 1.
Fig. 3 is a graph that illustrates a separation status by the separation method of Comparative Example 2.

### Description of Embodiments

The present invention relates to a method of separation and purification of a target cyclic macrolide compound from a mixture comprising the cyclic macrolide compound and at least one analogous compound thereof by silica gel column chromatography using an asymmetry identifying agent-immobilized silica gel.

The cyclic macrolide compound is tacrolimus.

Examples of the analogous compound of the cyclic macrolide compound include an analogous compound contained in a cultured product when the cyclic macrolide compound is produced by a culture technique. Examples thereof include compounds having structural differences from the cyclic macrolide compound, which lie in whether a double bond is contained in a side chain or not and the number of carbon atoms in the structure of 1 or 2. Examples of analogous compound of tacrolimus include ascomycin (FK520), FR-901156 and FR-900525. Structures of tacrolimus, ascomycin, FR-901156 and FR-900525 are shown in the following.

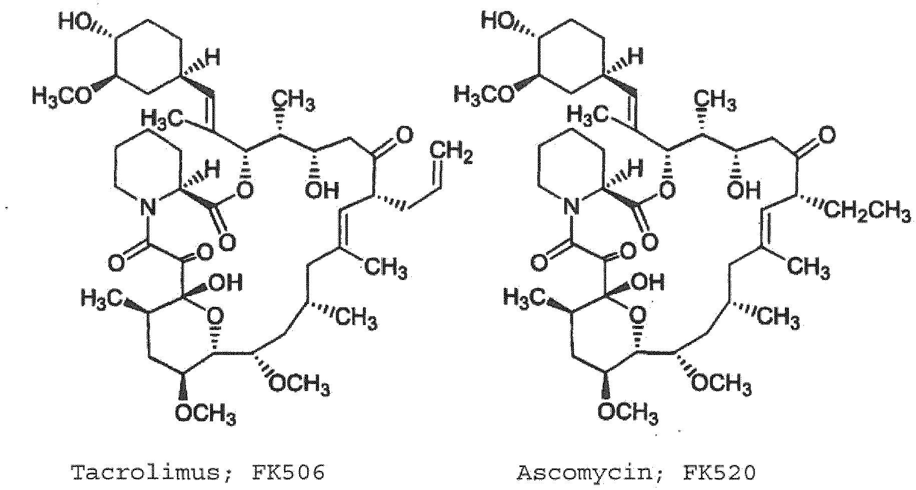

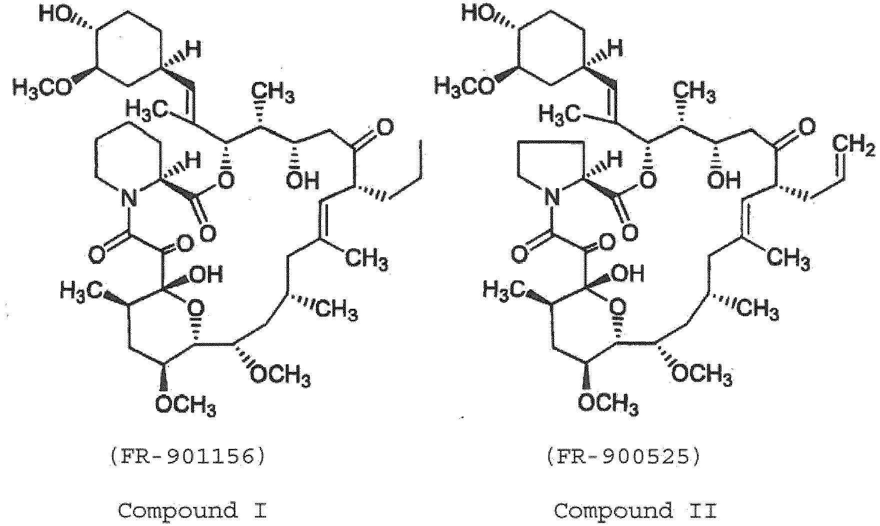

A mixture comprising a cyclic macrolide compound and at least one analogous compound thereof used in the present invention is preferably a mixture comprising tacrolimus and at least one selected from the group consisting of ascomycin, FR-901156 and FR-900525. More specifically, preferable is a crude purified product of a cultured solution containing such a mixture.

The mixture preferably contains 60% by mass (hereinafter simply referred to as %) or more of tacrolimus, more preferably contains 70% or more, and even more preferably contains 80% or more. In addition, the upper limit of the content of tacrolimus is preferably 98% or less, more preferably 95% or less, and even more preferably 90% or less. A specific content of tacrolimus in the mixture is preferably from 60 to 90%, more preferably from 70 to 95%, and even more preferably from 80 to 98%. The total amount of the analogous compound in the mixture is preferably 2% or more, more preferably 4% or more, further more preferably 5% or more. The analogous compound is preferably contained in the mixture preferably in an amount from 2 to 10%, more preferably from 4 to 10%, even more preferably from 5 to 10%.

An example of the mixture includes a crude purified product of a cultured product of tacrolimus producing microorganisms. Examples of the tacrolimus producing microorganisms include microorganisms described in JP-B No. 3-38276, for instance, tacrolimus producing microorganisms that belong to Streptomyces, tacrolimus producing microorganisms that belong to tacrolimus tsukubaensis belonging to streptomyces tsukubaensis, Streptomyces tsukubaensis No. 9993, and the like. Examples of the crude purified product include crude purified products described in the official gazette.

An asymmetry identifying agent-immobilized silica gel column is used as a separation column in the present invention. Tacrol imus and an analogous compound thereof are not enantiomers, but are compounds having different structures. Regardless of the fact, it is unexpected that tacrolimus and analogous compound thereof can be efficiently separated.

Examples of an asymmetry identifying agent-immobilized silica gel include a carbamate modified polysaccharides-immobilized silica gel, and an optically active amine modified synthetic polymer-immobilized silica gel. Examples of carbamate modified polysaccharides include carbamate modified cellulose, carbamate modified amylose, carbamate modified agarose, carbamate modified amylopectin, and carbamate modified cyclodextrin. More specific examples include phenyl carbamate modified polysaccharides represented by the formula (1) described below: 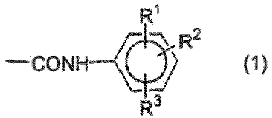 (wherein R¹, R² and R³ are the same or different and represent a hydrogen atom, a halogen atom, an alkyl group, an alkenyl group, an alkynyl group, an alkoxy group, an aryl group or an alkanoyl group.) Herein, an alkyl group having 1 to 6 carbon atoms is preferable as the alkyl group, an alkenyl group and an alkynyl group each having 2 to 6 carbon atoms are preferable as the alkenyl group and the alkynyl group. An alkoxy group having 1 to 6 carbon atoms is preferable as the alkoxy group. A phenyl group is preferable as the aryl group. An alkanoyl group having 2 to 6 carbon atoms is preferable as the alkanoyl group.

Examples of the optically active amines include (R) or (S) naphthylethylamine and (R) or (S) phenylethylamine.

The separation method of the present invention can be carried out in the same manner as in general silica gel column chromatography except for using the above described asymmetry identifying agent-immobilized silica gel column as a column. For example, a mixture comprising a cyclic macrolide compound and analogous compound thereof is passed through an asymmetry identifying agent-immobilized silica gel column and subsequently may be eluted with an eluent useful for elution of the compounds. Preferably, a mixed solution of saturated hydrocarbon such as hexane, alcohols and/or ethers, a mixed solution of acetonitrile, alcohol, ethers, water, and the like are used as an eluent.

The purity of tacrolimus obtained according to the method of the present invention is preferably 99.0% or more, more preferably 99.5% or more, and even more preferably 99.7% or more. Each content of the compound I and the compound II is preferably 0.1% or less, and the total amount of analogous compounds of ascomycin, the compound I and the compound II is preferably less than 0.27%.

### Examples

The details of the present invention will be explained with reference to examples.

### Reference Example 1

A medium (150 mL, pH 6.5) containing glycerine (1%), cornstarch (1%), glucose (0.5%), cotton seed powder (1%), corn steep liquor (0.5%), dried yeast (0.5%) and calcium carbonate (0.2%) is sterilized at 120°C for 30 minutes. The sterilized medium is inoculated with one inoculating loop of a slant cultured product of the Streptomyces tsukubaensis No. 9993 strain (the independent administrative corporation, The National Institute of Advanced Industrial Science and Technology, International Patent Organism Depositary, accession number FFRM BP-927) and shake culture is conducted at 30°C for 4 days. A medium (15 L, pH 6.8) containing soluble starch (4.5%), corn steep liquor (1.0%), dried yeast (1%), calcium carbonate (0.1%) and Adekanol (0.1%) is inoculated with the above obtained cultured product and cultured at 30°C for 4 days.

Activated carbon and diatom earth are added to the obtained culture broth and filtered. A mycelium mixture is extracted with 8 L of acetone to concentrate the extract. 170 g of sodium chloride and 1. 7 L of ethyl acetate are added to the concentrated product and, after stirring, the aqueous layer is removed. The organic layer is washed with an aqueous sodium chloride solution and the ethyl acetate layer is then concentrated. The obtained concentrated product is subjected to silica gel column chromatography, and fractions of tacrolimus eluted with mixed solutions of ethyl acetate : n-hexane of (1:1) and (4:1) are concentrated to thus obtain a mixture comprising tacrolimus, ascomycin, 17-propyl-1,14-dihydroxy-12-[2-(4-hydroxy-3-methoxycyclohex yl)-1-methylvinyl]-23,25-dimethoxy-13,19,21,27-tetramethyl-11,28-dioxa-4-azatricyclo[22.3.1.0^{4,9}]octacos-18-ene-2,3,10, 16-tetraone (hereinafter abbreviated as the compound I), and 16-allyl-1,13-dihydroxy-11-[2-(4-hydroxy-3-methoxycyclohexy 1)-1-methylvinyl]-22,24-dimethoxy-12,18,20,26-tetramethyl-1 0,27-dioxa-4-azatricyclo[21.3.1.0^{4,8}]heptacos-17-ene-2,3,9,1 5-tetraone (hereinafter abbreviated as the compound II). The composition ratio is 90.5% of tacrolimus, 6.4% of ascomycin, 2.6% of the compound I and 0.4% of the compound II.

A lower sum of the concentrations of ascomycin, the compound I and the compound II, which are contained in the separated active fraction, is better, and the sum is required to be less than 0.27% for practical uses.

### Example 1

### Separation by column chromatography using chiralpak (registered trademark) IC

4 mg of the mixture containing tacrolimus, ascomycin, the compound I and the compound II, which was obtained in the method shown in Reference Example 1, was subjected to column chromatography using 4 mL of Chiralpak (registered trademark) IC that is a silica gel obtained by chemically bonding tris(3,5-dichlorophenyl carbamate) cellulose being an asymmetry identifying agent, and eluted by use of a mixed solution of n-hexane : 2-propanol : methyl t-butyl ether (70:15:15) as an eluent.

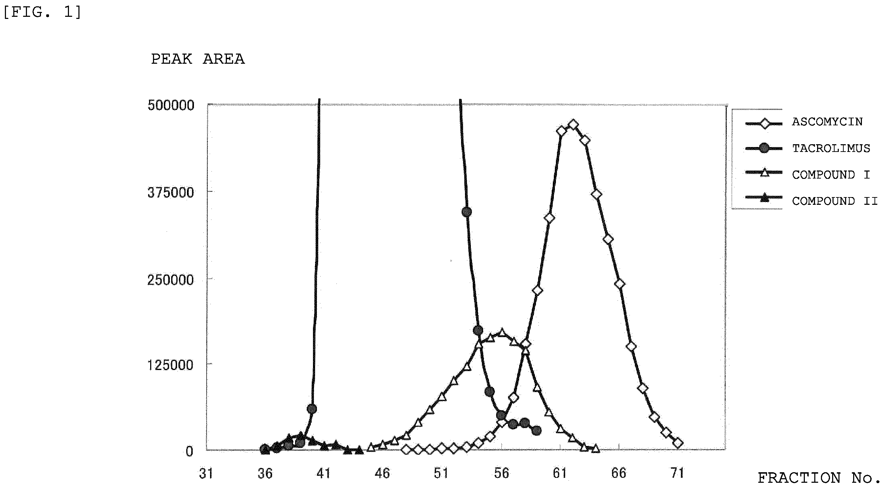

The result was shown in Fig. 1. The purity of the separated active fraction was measured by HPLC analysis. In the composition of the eluted fraction, tacrolimus was 99.88%, ascomycin was 0.08%, the compound I was 0.04%, and the compound II was not detected.

### Example 2

### Separation by column chromatography using Chiralpak (registered trademark) IE

In the same manner as in Example 1, 4 mg of a mixture comprising tacrolimus, ascomycin, the compound I and the compound II was subj ected to column chromatography using 4 mL of Chiralpak (registered trademark) IE that is a silica gel obtained by chemically bonding tris (3,5-dichlorophenyl carbamate) amylose being an asymmetry identifying agent, and eluted by use of a mixed solution of n-hexane : 2-propanol : methyl t-butyl ether (45:15:40) as an eluent.

The purity of the separated active fraction was measured by HPLC analysis. In the composition of the eluted fraction, tacrolimus was 100.00%, and all of ascomycin, the compound I and the compound II were not detected.

### Comparative Example 1

### Separation by column chromatography using DIAION (registered trademark) HP20SS and silver nitrate-containing eluent

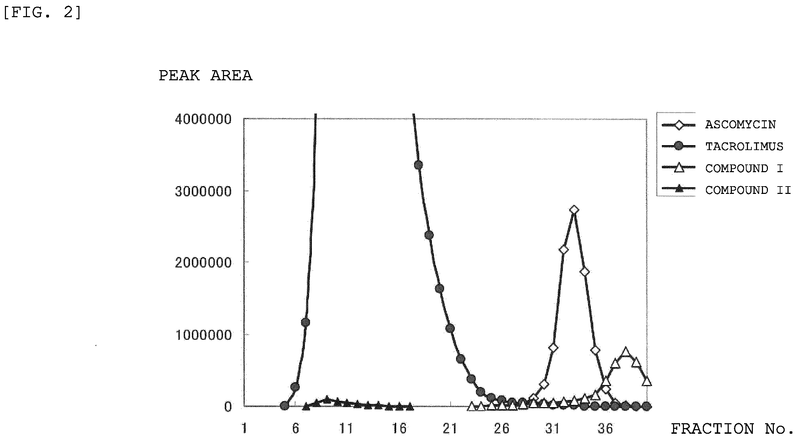

In the same manner as in Example 1, 200 mg of a mixture comprising tacrolimus, ascomycin, the compound I and the compound II was subjected to column chromatography using 20 mL of DIAION (registered trademark) HP20SS, and eluted by use of a 0.294 mol/L-silver nitrate-containing aqueous 50% acetone solution and subsequently an aqueous 60% acetone solution as eluents. The result was shown in Fig. 2. The purity of the separated active fraction was measured by HPLC analysis. In the composition of the eluted fraction, tacrolimus was 99.73%, ascomycin and the compound I were not detected, and the compound II was 0.27%.

### Comparative Example 2

### Separation by column chromatography using silver salt of DIAION (registered trademark) RCP 160M ion exchange resin

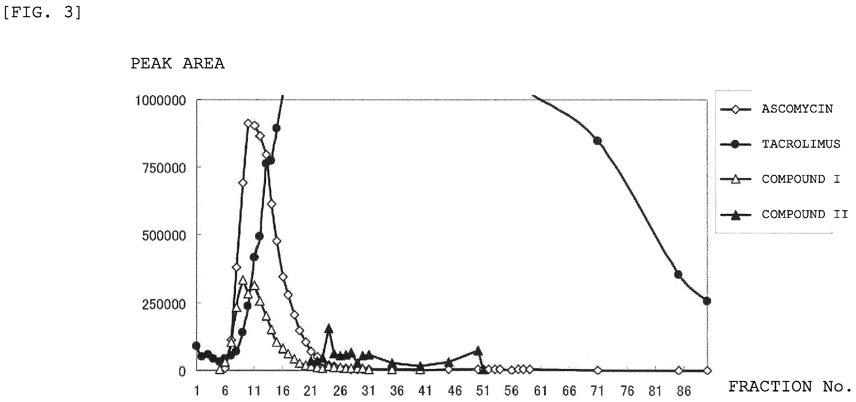

15.6 mL of DIAION (registered trademark) RCP 160M ion exchange resin was adsorbed with silver ions by a silver nitrate solution and, in the same manner as in Example 1, 200 mg of a mixture comprising tacrolimus, ascomycin, the compound I and the compound II was then added thereto and eluted by use of a mixed solution of ethyl acetate : methanol (1:) as an eluent. The result was shown in Fig. 3. The purity of the separated active fraction was measured by HPLC analysis. In the composition of the eluted fraction, tacrolimus was 99.35%, ascomycin was 0.2%, the compound I was 0.06%, and the compound II was 0.4%.

### Example 3

### Separation by column chromatography using YMC Chiral NEA (registered trademark) (R)

In the same manner as in Example 1, 1 mg of a mixture of tacrolimus, ascomycin, the compound I and the compound II was subjected to column chromatography using 2.5 mL of Chiral NEA (registered trademark) (R) that is a silica gel on which a methacrylate polymer obtained by bonding (R)-1-(α-naphthyl)ethylamine being an asymmetry identifying agent is immobilized, and eluted by use of 35% acetonitrile as an eluent. The purity of the separated active fraction was measured by HPLC analysis. In the composition of the eluted fraction, tacrolimus was 99.94%, ascomycin was 0.02%, the compound I was not detected, and the compound II was 0.04%.

## Claims

1. A method for separating a cyclic macrolide compound from a mixture comprising a cyclic macrolide compound and at least one analogous compound thereof, wherein the mixture is subjected to silica gel column chromatography using an asymmetry identifying agent- immobilized silica gel, wherein the cyclic macrolide compound is tacrolimus and the asymmetry identifying agent-immobilized silica gel is a carbamate modified polysaccharide-immobilized silica gel or an optically active amine modified synthetic polymer-immobilized silica gel.

2. The method according to claim 1, wherein the analogous compound is at least one selected from the group consisting of ascomycin, FR-901156 and FR-900525.

3. A method for producing a cyclic macrolide compound comprising an analogous compound thereof in the total amount of less than 0.27%, wherein a mixture comprising a cyclic macrolide compound and at least one analogous compound thereof is subjected to silica gel column chromatography using an asymmetry identifying agent-immobilized silica gel, wherein the cyclic macrolide compound is tacrolimus and the asymmetry identifying agent-immobilized silica gel is a carbamate modified polysaccharide-immobilized silica gel or an optically active amine modified synthetic polymer-immobilized silica gel.

4. The method according to claim 3, wherein the cyclic macrolide compound is tacrolimus and the analogous compound thereof is at least one selected from the group consisting of ascomycin, FR-901156 and FR-900525.

## Patentansprüche

1. Methode zur Abtrennung einer zyklischen Makrolidverbindung aus einer Mischung enthaltend eine zyklische Makrolidverbindung und mindestens eine analoge Verbindung derselben, wobei die Mischung einer Silikagel-Säulenchromatographie unterzogen wird unter Verwendung eines Silikagels mit einem immobilisierten Asymmetrie-identifizierenden Mittel, wobei die zyklische Makolidverbindung Tacrolimus ist und das Silikagel mit einem immobilisierten Asymmetrie-identifizierenden Mittel ein Silikagel ist mit einem immobilisierten Carbamat-modifizierten Polysaccharid oder ein Silikagel mit immobilisiertem mit einem optisch aktiven Amin modifizierten synthetischen Polymer ist.

2. Die Methode gemäß Anspruch 1, worin die analoge Verbindung mindestens eine ausgewählt aus der Gruppe bestehend aus Ascomycin, FR-901156 und FR-900525 ist.

3. Methode zur Herstellung einer zyklischen Makrolidverbindung enthaltend eine analoge Verbindung derselben in der Gesamtmenge von weniger als 0,27 %, wobei eine Mischung enthaltend eine zyklische Makrolidverbindung und mindestens eine analoge Verbindung derselben einer Silikagel-Säulenchromatographie unterzogen wird unter Verwerdung eines Silikagels mit einem immobilisierten Asymmetrie-identifizierenden Mittel, wobei die zyklische Makolidverbindung Tacrolimus ist und das Silikagel mit einem immobilisierten Asymmetrie-identifizierenden Mittel ein Silikagel ist mit einem immobilisierten Carbamat-modifizierten Polysaccharid oder ein Silikagel mit immobilisiertem mit einem optisch aktiven Amin modifizierten synthetischen Polymer ist.

4. Die Methode gemäß Anspruch 3, worin die zyklische Makrolidverbindung Tacrolimus ist und die analoge Verbindung derselben mindestens eine ausgewählt aus der Gruppe bestehend aus Ascomycin, FR-901156 und FR-900525 ist.

## Revendications

1. Procédé pour séparer un composé macrolide cyclique à partir d'un mélange comprenant un composé macrolide cyclique et au moins un composé analogue de celui-ci, dans lequel le mélange est soumis à une chromatographie sur colonne de gel de silice utilisant un gel de silice sur lequel est immobilisé un agent identificateur d'asymétrie, et dans lequel le composé macrolide cyclique est le tacrolimus et le gel de silice sur lequel est immobilisé un agent identificateur d'asymétrie est un gel de silice sur lequel est immobilisé un polysaccharide modifié par un carbamate ou un gel de silice sur lequel est immobilisé un polymère synthétique modifié par une amine optiquement active.

2. Procédé selon la revendication 1, dans lequel le composé analogue est au moins l'un choisi dans l'ensemble constitué par l'ascomycine, FR-901156 et FR-900525.

3. Procédé pour produire un composé macrolide cyclique comprenant un composé analogue de celui-ci en une quantité totale inférieure à 0,27 %, dans lequel un mélange comprenant un composé macrolide cyclique et au moins un composé analogue de celui-ci est soumis à une chromatographie sur colonne de gel de silice utilisant un gel de silice sur lequel est immobilisé un agent identificateur d'asymétrie, et dans lequel le composé macrolide cyclique est le tacrolimus et le gel de silice sur lequel est immobilisé un agent identificateur d'asymétrie est un gel de silice sur lequel est immobilisé un polysaccharide modifié par un carbamate ou un gel de silice sur lequel est immobilisé un polymère synthétique modifié par une amine optiquement active.

4. Procédé selon la revendication 3, dans lequel le composé macrolide cyclique est le tacrolimus et le composé analogue de celui-ci est au moins l'un choisi dans l'ensemble constitué par l'ascomycine, FR-901156 et FR-900525.
